(19) Europäisches Patentamt
European Patent Office
Office européen des brevets

(11) EP 0 282 287 B2

(12) NEW EUROPEAN PATENT SPECIFICATION

(45) Date of publication and mention
of the opposition decision:
**24.04.1996 Bulletin 1996/17**

(45) Mention of the grant of the patent:
**25.03.1992 Bulletin 1992/13**

(21) Application number: **88302054.7**

(22) Date of filing: **09.03.1988**

(51) Int. Cl.$^6$: **A61L 9/01**, A61L 15/00

(54) **Deodorizer**

Desodorierungsmittel

Désodorisant

(84) Designated Contracting States:
**AT BE CH DE ES FR GB IT LI NL SE**

(30) Priority: **10.03.1987 JP 54860/87**
**30.06.1987 JP 162681/87**
**28.12.1987 JP 332326/87**

(43) Date of publication of application:
**14.09.1988 Bulletin 1988/37**

(73) Proprietor: **LION CORPORATION**
**Sumida-ku Tokyo (JP)**

(72) Inventors:
• **1 Yamada, Kohichi**
**Sakura-shi Chiba (JP)**
• **Ueno, Akira**
**Yachiyo-shi Chiba (JP)**
• **Sato, Teiji**
**Shibata-shi Niigata (JP)**
• **Kuroda, Hideo**
**Kawasaki-shi Kanagawa (JP)**
• **Iwamoto, Miyako**
**Yokohama-shi Kanagawa (JP)**
• **Tanaka, Masanori**
**Shibata-shi Niigata (JP)**
• **Ishimatsu, Tetsuo**
**Funabashi-shi Chiba (JP)**

(74) Representative: **Adams, William Gordon et al**
**RAWORTH, MOSS & COOK**
**36 Sydenham Road**
**Croydon Surrey CR0 2EF (GB)**

(56) References cited:
WO-A-81/01643          GB-A-  514 979
GB-B- 1 429 143       US-A- 2 634 229
US-A- 3 340 875       US-A- 3 669 109
US-A- 3 939 838       US-A- 4 257 885
US-A- 4 525 410       US-A- 4 636 373

• Encyclop. of Chem. Technology, 3rd ed. John
Wiley & Sons, 1979, vol. 7, 15,22.
• Kirk Othmer, Encycl. of Chem. Technol., vol. 12
pages 268,288,282.

Remarks:
The file contains technical information submitted
after the application was filed and not included in this
specification

EP 0 282 287 B2

EP 0 282 287 B2

**Description**

BACKGROUND OF THE INVENTION

1. Field of the Invention

The present invention relates to a deodorizer. More specifically, it relates to a deodorizer capable of effectively removing various offensive or undesirable smells derived from, for example, hydrogen sulfide, ammonia, mercaptans, amines, lower fatter acids. The present invention also relates to sanitary goods for absorbing a humor, such as sanitary napkins and baby diapers and a deodrizing sheet.

2. Description of the Related Art

The increasing urbantization of formerly sparsely-populated areas has brought more people into daily contact with the various smells and odors of their environment and this has led to a strong interest in and severe criticism of particularly offensive odors. Various methods for treating offensive odors are known in the art, of which the following are typical:

(1) Sensitive Deodorizing ... Masking with perfumes or flavors;
(2) Physical Deodorizing ... Adsorption with, for example, activated carbon or absorption or clathrating with, for example, cyclodextrin;
(3) Chemical Deodorizing ... Neutralization with an acid or alkali, oxidation or reduction with an oxidizing agent or reducing agent, or addition with, for example, lauryl methacrate; and
(4) Biological Deodorizing ... deodorizing by germicidal action with germicide, or effects with microorganisms or enzymes.

The sensitive deodorizing method is often unsuccessful, since only the flavor of the ordor is changed and the offensive odor itself is still present although masked, and thus if the balance between the offensive odor and the flavor is lost, the offensive odor again prevails and the balance must be restored.

In the physical deodorizing method, although the offensive odor is, absorped or clathrated, problems arise in that the adsorption power or clathrating power is not strong enough for the practical use.

In the chemical deodorizing method, certain chemicals should not be used from the viewpoint of safety and a chemical deodorization of one offensive odor can be achieved, this same process will have no affect on the other various offensive odors generated in daily life.

In the biological deodorizing method, disadvantages in the deodorizing rate and a continuous effect or durability arise and, therefore, universal effects cannot be obtained by a single deodorizing method.

Conventionally activated carbon is most widely used, and is known as a deodorizer capable of adsorbing various offensive odor components. However, of these offensive odor components, the capability of activated carbon for the adsorption of lower amines is small and, in particular, the deodorizing power thereof against hydrogen sulfide and ammonia is low.

Various attempts have been made to solve the above-mentioned problems. For example, JP-A-55-51421 (i.e., Japanese Unexamined Patent Publication) proposes that halides be supported on activated carbon; JP-A-53-137089 proposes that metals be supported on activated carbon; and the adhesion or deposition of acids or alkalis has been studied.

However, since these activated carbons exhibit acid or alkaline properties when moisture or the like is adhered thereto, they cause unpreferable corrosion or must be handled as a hazardous material and, therefore, are not suitable for use in daily life.

Furthermore, it is disclosed in, for example, JP-A-58-156539 and 59-146578, that ferrous or ferric salts can be used as a deodorizer. Especially, it is reported that ferrous salts are effective for deodorizing basic or alkaline offensive odors such as ammonia, but can not cope with hydrogen sulfide, mercaptan or the like.

Furthermore, various sanitary goods for absorbing a humor, such as sanitary napkins and paper diapers having absorbents for liquid provided inside, have been proposed. Basically, these sanitary goods must, of course, have an excellent absorption power, but in addition to the absorption power, it is also important that they suppress the offensive odors derived from the humor. For example, offensive odors are generated from sanitary napkins which have absorbed menstrual blood. These odors are generated from the formation of, for example, ammonia, amines, mercaptans, and hydrogen sulfide by a decomposition of amino acids contained in the menstrual blood. To suppress these odors, conventionally activated carbon is most widely used, but since activated carbon is black, it is not preferable for use with such sanitary goods from the standpoint of visual appearance. Many proposals have been made to use non-black deodorizers such as zeolites and chlorophyll, as disclosed in, for example, Japanese Unexamined Utility Model Publication Nos. 48-115995, 49-6898, 52-86299, 55-75318, and 60-8249803, but sanitary goods having satisfactory deodorizing or odor-preventing effects can not be obtained thereby.

2

Furthermore, various deodorizing sheets are utilizaed in, for example, filter materials, sanitary goods, and other deodorizer in the form of a sheet. Conventionally, the deodorizing sheets are prepared by adhering activated carbon to a porous sheet an adhesive, or by sheeting a blend of fiber and activated carbon as disclosed in Japanese Unexamined Patent Publication Nos. 53-113288 and 53-61582. However, again, the use of activated carbon is not preferable from the standpoint of visual appearance as mentioned above. In addition, since there are certain selectivities in the gas absorbability of activated carbon, the wide effects for various odors cannot be expected.

## SUMMARY OF THE INVENTION

Accordingly, the objects of the present invention are to eliminate the above-mentioned disadvantages of the prior art and to provide a deodorizer capable of exhibiting a strong and excellent deodorizing power against various offensive odors caused by, for example, hydrogen sulfide, ammonia, mercaptans, amines, and lower fatty acids generated in daily life, and having a high safety and easy handling properties.

Another object of the present invention is to provide sanitary goods having excellent deodorizing or odor-preventing effects against unpleasant odors generated from a humor, especially a menstrual odor, excellent appearance because the color black is not used, and are comfortable to wear when in use.

A further object of the present invention is to provide a white deodorizing sheet having excellent deodorizing effects against a wide variety of odors.

Other objects and advantages of the present invention will be apparent from the following description.

The present invention provides a method of making a deodorizer as defined in the appended claims.

The invention extends to sanitary goods and a deodorizing sheet comprising such a deodorizer.

## BRIEF DESCRIPTION OF THE DRAWING

The present invention will be better understood from the description set forth below with reference to the drawings, in which:

Figure 1 illustrates an apparatus for determining the deodorizing amounts; and

Figure 2 illustrates a test method for determining the deodorizing effects.

## DESCRIPTION OF THE PREFERRED EMBODIMENTS

The metal silicates and metal aluminum-containing silicates made according to the present invention have the above-mentioned composition, in terms of an oxide. The preferable compositions are as follows:

| | |
|---|---|
| $SiO_2$: | 25 to 75 mole% |
| $MOn_{/2}$; | 15 to 60 mole% |
| $Al_2O_3$: | 0 to 45 mole% |

They are usually in the form of a white or pale-colored powder and are obtained by reacting water-soluble silicates, water-soluble metal salts and, if appropriate, water-soluble aluminum salts and/or water-soluble aluminates, in an amount ratio corresponding to the above-mentioned composition ratio in the presence of water, followed by, if necessary, heating the resultant precipitates in the presence of water.

The above-mentioned reaction readily proceeds by a metathetical reaction. That is, when an alkali silicate such as sodium silicate is used as a silica component, when a water-soluble metal salt such as chloride, nitrate, or sulfate is used as a metal oxide component, and optionally, when sodium aluminate and/or a water-soluble aluminum salt such as aluminum chloride or aluminum sulfate are used if alumina is to be included, these components are mixed together in the presence of water, followed by effecting the metathetical reaction.

In the case of sanitary goods, a white inorganic powder having oxide compositions of 5 to 60 mole%, preferably 15 to 55 mole% of ZnO, 5 to 80 mole%, preferably 25 to 75 mola% of $SiO_2$, and 0 to 60 mole%, preferably 0 to 45 mole% of $Al_2O_3$ is used as a deodorizer. These materials are obtained by reacting water-soluble silicates, water-soluble zinc salts, and optionally, water-soluble aluminum salts and/or water-soluble aluminates, in an amount ratio corresponding to the above-mentioned composition ratio, in the presence of water, followed by, if necessary, heating the resultant precipitates in the presence of water. This is also the metathetical reaction as mentioned above. Thus, when an alkali silicate such as sodium silicate is used as a silica component, when a zinc salt such as zinc chloride, nitrate, or sulfate is used as a ZnO component, and optionally when sodium aluminate and/or a water-soluble aluminum salt such as aluminum chloride or aluminum sulfate are used if alumina is to be included, these components are mixed together in the presence of water, followed by effect the metathetical reaction.

To uniformly effect the above-mentioned metathetical reactions, it is preferable to simultaneously add, to an aqueous dispersion containing silica previously dispersed therein, an aqueous silicate solution, an aqueous metal salt solution (or an aqueous zinc salt solution in the case of the sanitary goods) and, optionally, an aqueous solution containing the alumina component, while allowing a reaction. The metal silicates or metal aluminum-containing silicates obtained by this method have superior deodorizing effects.

The above-mentioned metathetical reaction can be carried out at room temperature, but also can be carried out upon heating at a temperature of up to, for example, about 95°C. The preferable pH range of the reaction system during the simultaneous addition is preferably maintained at 5 to 10, especially 6 to 9. If necessary, the pH of the reaction mixture can be controlled by the addition of an acid or alkali to maintain the above-mentioned pH range.

The above-mentioned simultaneous addition of the reactants allows to the formation of precipitated metal salts (or zinc salt) of silicates or metal salts (or zinc salts) of aluminum-containing silicate having a composition substantially corresponding to those of the aqueous solutions. The precipitates thus formed are separated, or optionally are heated in the presence of water, to obtain white or pale-colored fine powder (or white fine powder in the case of zinc silicate).

Alternatively, metal silicates or metal aluminum-containing silicates can be produced by co-precipitating an aqueous solution containing the metal salts or the aluminum salts therewith under an alkaline condition, followed by hydrothermally reacting the resultant precipitates and silica under pressure.

Furthermore, in the case of the zinc silicates or zinc aluminum-containing silicates, the desired silicates can be produced by co-precipitating an aqueous solution containing zinc salts or the aluminum salts therewith under an alkaline condition, followed by hydrothermally reacting the resultant precipitates and silica under pressure.

The resultant product can be dried at a temperature of, for example, 100°C or more, preferably 150°C to 220°C. Thus, the desired white powder can be obtained in the case of zinc silicates or zinc aluminum-containing silicates. Although there are no critical limitations to the particle sizes of the resultant powder, the average diameter is preferably 0.1 to 100 $\mu$m, more preferably 0.5 to 30 $\mu$m.

Although there are no critical limitations to the surface area of the silicates, the preferable specific surface area of 100 $m^2$/g or more, determined according to a BET method using nitogen.

Since the present deodorizer contains, as an effective component, the above-mentioned metal silicates or metal aluminum-containing silicate, the deodorizer possesses the characteristics of both a solid acid and a solid bases. Furthermore, in the structure of the present deodorizer, the acidic $SiO_2$ and the basic metal oxide are bonded, and accordingly, the present deodorizer exhibits good deodorizing effects against both basic offensive odors and acidic offensive odors.

Although the deodorizing mechanism of the present deodorizer composition is not clear, the oxidation and reduction reactions caused by the above-mentioned structure and the adsorption function of the metal silicates or the metal aluminium-containing silicates are synergistically combined.

Since the metal silicates or the metal aluminum-containing silicates used as an effective component in the present deodorizer composition can be obtained in the form of a fine powder, the present deodorizer can be readily supported on, for example, paper or sheet, and has an excellent processability or workability. Thus, the present deodorizer has a high applicability.

Furthermore, since the present deodorizer composition uses the metal aluminum-containing silicate comprising $SiO_2$, ZnO, $Al_2O_3$, and $H_2O$, the present deodorizer composition is non-toxic and has a high safety. Furthermore, the present deodorizer can be appropriately used in the fields of, for example, cosmetics, sanitary napkins, and paper diapers, due to the high whiteness, thereof.

When the present deodorizer is used as sanitary goods, the present deodorizer is used together with polymer absorbents. The polymer absorbents usable in the present invention are those which are water-insoluble and water-swellable. Examples of such polymer absorbents are homopolymers or copolymers containing, as a fundamental constituent, acrylic acid and methacrylic acid such as crosslinked sodium polyacrylates and crosslinked sodium polymethacrylates. The preferable average size of the polymer absorbent powder is 10 to 1000 $\mu$m, more preferably 150 to 600 $\mu$m.

In sanitary goods according to the present invention, the above-mentioned inorganic white powder is used in combination with the above-mentioned polymer absorbent powder. Although there are no critical limitations to the ratio of the inorganic white powder and the polymer absorbent powder to be used in the sanitary goods, the preferable amount of the inorganic white powder is 5 to 80 parts by weight, more preferably 10 to 50 parts by weight, based on 100 parts by weight of the polymer absorbent powder.

The inorganic white powder and the polymer absorbent can be combined in any form. That is, both components can be mixed together, or both components can be separately placed, for example, the inorganic white powder layer and the polymer absorbent layer can be combined as independent layers. Furthermore, the inorganic white powder can be coated on the surface of the polymer absorbent powder. The inorganic white powder and the polymer absorbent powder can be supported at any part of the sanitary goods. For example, these powders can be dispersed in the absorbent for sanitary goods, can be located on the surface of the raw materials such as non-woven fabrics covering the absorbent, or can be inserted into, or laminated on the surface of, the absorbent in the form of a layer. In the case of, for example, sanitary napkins or paper diapers, these powders are preferably laminated in the absorbent.

According to the present invention, the deodorizing sheet containing white zinc aluminosilicate is provided as mentioned above.

The zinc aluminosilicate in the form of white powder is prepared by reacting water-soluble silicates, water-soluble zinc salts, and water-soluble aluminum salts and/or water-soluble aluminates, in an amount ratio corresponding to the above-mentioned composition ratio in the presence of water, followed by, if necessary, heating the resultant precipitates in the presence of water.

The above-mentioned reaction readily proceeds by a metathetical reaction. That is, when an alkali silicate such as sodium silicate is used as a silica component, when a water-soluble zinc salt such as the chloride, nitrate or sulfate is used as a metal oxide component, and, when sodium aluminate and/or a water-soluble aluminum salt such as aluminum chloride or aluminum sulfate are used, these components are mixed together in the presence of water, followed by effecting the metathetical reaction. The metathetical reaction conditions may be the same as mentioned above. As the zinc aluminosilicate, those having a Hunter's whiteness of 80% or more are preferably used, because the visual appearance can be further improved.

As the porous sheet materials usable in the present deodorizing sheets, natural or regenerated fiber such as cotton, rayon, pulp, linen; and synthetic or semi-synthetic fiber such as acetate, polyester, and polyamide may be used. The zinc aluminosilicate may be dispersed or supported on the porous sheet in any conventional manner. For example, a binder is used, or a sheet is made by blending with fiber such as short cut fiber of, for example, pulp, rayon, polypropylene fiber, or ester fiber. The zinc aluminosilicate powder may be added to disperse in a dispersion, followed by making a deodorizing sheet.

There are no critical limitations to the amount of the zinc aluminosilicate, the zinc aluminosilicate is preferably dispersed or supported on the porous sheet in an amount of 1% to 50% by weight, based on the total weight of the deodorizing sheet. When the amount of zinc aluminosilicate is too small, the deodorizing effects are decreased. Contrary to this, when the amount of zinc aluminosilicate is too large, the zinc aluminosilicate tend to be removed or separated from the surface of the porous sheet.

As explained above and in Examples hereinbelow, according to the present invention, excellent deodorizing effects against various offensive odors generated from such as hydrogen sulfide, mercaptans, amines, and lower fatty acids can be obtained by using the metal silicates or metal aluminum containing silicates having the specified compositions.

Furthermore, according to the present invention, sanitary goods capable of effectively suppressing unpleasant odors generated from the absorbed humor and having an excellent appearance due to the whiteness of the inorganic white powder, and providing more comfort when worn, compared with those using a colored deodorizing component such as activated carbon, can be obtained, and thus the effects of the present invention are remarkable in women's sanitary napkins and will be greatly appreciated by women.

Furthermore, since the zinc aluminosilicate posseses a basic structure in which a $SiO_4$ or $AlO_4$ tetrahedron layer and a $ZnO_6$ octahedron layer are combined in two or three layers, the zinc aluminosilicate has the properties of a solid acid or solid base and acts effectively against a variety of offensive odors. Consequently, the deodorizing sheet of the present invention exhibits an excellent deodorizing power against various offensive or unpleasant odors and also has a high degree of whiteness and therefore, is widely applicable as, for example, a deodorizing sheet in sanitary goods, filters, and the like.

EXAMPLE

The present invention will now be further, illustrated by, but is by no means limited to, the following Examples.

Example 1

A 109 g amount of #3 sodium silicate ($SiO_2$: 22.0%, $Na_2O$: 7.0%) and 94 g of sodium hydroxide (NaOH content: 2.35 mole) were dissolved in water to prepare 1 liter of a solution A ($SiO_2$ content: 0.4 mole).

On the other hand, 95 g of zinc chloride (anhydrous salt) and 97 g of aluminum chloride (hexahydrate) were dissolved in water to prepare 1 liter of a solution B (ZnO content: 0.7 mole, $Al_2O_3$: 0.2 mole).

One liter of water was charged into a 5 liter beaker and, while stirring, the solutions A and B were simultaneously added thereto at a feed rate of about 25 cc/min, respectively. The pH of the resultant reaction mixture was 6.9 after the addition.

The stirring was further continued and, after aging for 30 minutes, the mixture was heated at a temperature of 85 to 90°C for 2 hours on a water bath. The reaction mixture thus obtained was filtered with aspiration, followed by washing with water and drying at a temperature of 110°C. The cake thus obtained was shifted with a sieve to obtain metal (zinc) salt of aluminum-containing silicate in the form of white particles having size of 8 to 16 mesh (Tylor).

The composition of the three components and the BET specific surface of the resultant particles are shown in Table 2 together with the data of the following Examples and Comparative Examples after Comparative Example 3.

Example 2

A 139 g amount of #3 sodium silicate ($SiO_2$: 22.0%, $Na_2O$: 7.0%) and 88 g of sodium hydroxide (NaOH content: 2.2 mole) were dissolved in water to prepare 1 liter of a solution A ($SiO_2$ content: 0.51 mole).

On the other hand, 65 g of zinc chloride (anhydrous salt) and 126 g of aluminum chloride (hexahydrate) were dissolved in water to prepare 1 liter of a solution B (ZnO content: 0.48 mole, $Al_2O_3$: 0.26 mole).

One liter of water was charged into a 5 liter beaker and, while stirring, the solutions A and B were simultaneously added thereto at a feed rate of about 25 cc/min, respectively. The pH of the resultant reaction mixture was 8.6 after the addition.

The reaction mixture obtained above was treated in the same manner as in Example 1. Thus, metal (zinc) salt of aluminum-containing silicate in the form of particles having a size of 8 to 16 meshes.

The results are shown in Table 1.

Example 3

A 164 g amount of #3 sodium silicate ($SiO_2$: 22.0%, $Na_2O$: 7.0%) and 57 g of sodium hydroxide (NaOH content: 1.43 mole) were dissolved in water to prepare 1 liter of a solution A ($SiO_2$ content: 0.6 mole).

On the other hand, 123 g of zinc chloride (anhydrous salt) was dissolved in water to prepare 1 liter of a solution B (ZnO content: 0.9 mole).

One liter of water was charged into a 5 liter beaker and, while stirring, the solutions A and B were simultaneously added thereto at a feed rate of about 25 cc/min, respectively. The pH of the resultant reaction mixture was 6.1 after the addition.

The reaction mixture obtained above was treated in the same manner as in Example 1. Thus, metal (zinc) silicate, in the form of particles having a size of 8 to 16 meshes.

The results are shown in Table 2.

Example 4

A 77 g amount of #1 sodium silicate ($SiO_2$: 35.0%, $Na_2O$: 17.5%) and 24 g of sodium hydroxide (NaOH content: 0.6 mole) were dissolved in water to prepare 1 liter of a solution A ($SiO_2$ content: 0.45 mole).

On the other hand, 216 g of zinc chloride (heptahydrate) was dissolved in water to prepare 1 liter of a solution B (ZnO content: 0.75 mole).

Furthermore, 75 g of sodium aluminate ($Al_2O_3$ content: 20.5%, $Na_2O$: 19.5%) was diluted with water to prepare 1 liter of a liquid C ($Al_2O_3$ content: 0.15 mole).

One liter of water was charged into a 5 liter beaker and, while stirring, the liquids A, B, and C were simultaneously added thereto at a feed rate of about 25 cc/min, respectively. The pH of the resultant reaction mixture was 7.0 after the addition.

The reaction mixture obtained above was treated in the same manner as in Example 1. Thus, metal (zinc) salt of aluminum-containing silicate in the form of particles having a size of 8 to 16 meshes.

The results are shown in Table 2.

Example 5

A 100 g amount of sodium hydroxide (NaOH content: 2.5 mole) was dissolved in water to prepare 0.4 liters of a solution A.

On the other hand, 109 g of zinc chloride (anhydrous salt) and 97 g of aluminum chloride (hexahydrate) were dissolved in water to prepare 1 liter of a solution B (ZnO content: 0.72 mole, $Al_2O_3$ content: 0.2 mole).

One liter of water was charged into a 3 liter beaker and, while stirring, the solutions A and B were simultaneously added thereto at a feed rate of about 10 cc/min, respectively. The pH of the resultant reaction mixture was 10.0 after the addition.

The stirring was further continued and, after aging for 30 minutes, the mixture was heated at a temperature of 90° C for 2 hours on a water bath. The reaction mixture thus obtained was filtered with aspiration, followed by washing with water.

The cake obtained above and 31 g of finely divided silica (Mizukasil P-526N manufactured by Mizusawa Chemical Co.) (dried at 110°C, $SiO_2$ content: 0.5 mole) were placed in a 1 liter autoclave, followed by adding water to make the total volume to 600 ml. The hydrothermal synthetic reaction was carried out at a temperature of 160°C for 3 hours under a stirring condition of 300 rpm. After cooling, the reaction product was collected and filtered to remove water, followed by drying at a temperature of 110°C. The resultant cake was shifted with a sieve to obtain metal (zinc) salt of aluminum-containing silicate in the form of white particles having a size of 8 to 16 mesh.

The results are shown in Table 2.

Example 6

A 205 g amount of #3 sodium silicate ($SiO_2$: 22.0%, $Na_2O$: 7.0%) and 221 g of sodium hydroxide (NaOH content: 5.5 mole) were dissolved in water to prepare 1 liter of a solution A ($SiO_2$ content: 0.75 mole).

On the other hand, 180 g of zinc chloride (anhydrous salt) and 241 g of aluminum chloride (six hydrates) were dissolved in water to prepare 1 liter of a solution B (ZnO content: 1.2 mole, $Al_2O_3$: 0.6 mole).

Furthermore, hydrogel prepared by neutralizing, under an acidic condition of pH of 2 to 4, 330 g of the #3 sodium silicate and about 80 g of hydrochloric acid (HCl: 35%) was crushed and slurried in a home mixer to obtain a silica dispersion ($SiO_2$ content: 4.8%).

A 1.5 kg amount of the silica dispersion ($SiO_2$ content: 1.2 mole) was charged into a 5 liter beaker and, while stirring, the solutions A and B were simultaneously added thereto at a feed rate of about 25 cc/min, respectively. The pH of the resultant reaction mixture was 7.2 after the addition.

The stirring was further continued and aged for one hour. The reaction mixture thus obtained was filtered with aspiration, followed by washing with water and drying at a temperature of 110° C. The cake thus obtained was shifted with a sieve to obtain metal (zinc) salt of aluminum-containing silicate in the form of particles having a size of 8 to 16 mesh.

The results are shown in Table 2.

Example 7

A 220 g amount of #3 sodium silicate ($SiO_2$: 22.0%, $Na_2O$: 7.0%) and 80 g of sodium hydroxide (NaOH content: 2 mole) were dissolved in water to prepare 1 liter of a solution A ($SiO_2$ content: 0.8 mole).

On the other hand, 180 g of zinc chloride (anhydrous salt) was dissolved in water to prepare 1 liter of a solution B (ZnO content: 1.2 mole).

Furthermore, hydrogel prepared by neutralizing, under an acidic condition of pH of 2 to 4, 275 g of the #3 sodium silicate and about 70 g of hydrochloric acid (HCl: 35%) was crushed and slurried in a home mixer to obtain a silica dispersion.

A 1.25 kg amount of the silica dispersion ($SiO_2$ content: 1.0 mole) was charged into a 5 liter glass vessel and, while stirring and maintaining the liquid temperature at 40°C, the solutions A and B were simultaneously added thereto at a feed rate of about 25 cc/min, respectively, the pH of the resultant reaction mixture was 7.3 after the addition.

The stirring was further continued and aged for one hour. The reaction mixture thus obtained was filtered with aspiration, followed by washing with water and drying at a temperature of 110°C. The cake thus obtained was shifted with a sieve to obtain metal (zinc) silicate in the form of particles having a size of 8 to 16 mesh.

The results are shown in Table 1.

Example 8

A 273 g amount of #3 sodium silicate ($SiO_2$: 22.0%, $Na_2O$: 7.0%) and 60 g of sodium hydroxide (NaOH content: 1.5 mole) were dissolved in water to prepare 1 liter of a solution A ($SiO_2$ content: 1.0 mole).

On the other hand, 34 g of silver nitrate and 225 g of aluminum nitrate (nonahidrate) were dissolved in water to prepare 1 liter of a solution B ($Ag_2O$ content: 0.1 mole, $Al_2O_3$ content: 0.3 mole).

One liter of water was charged into 5 liters beaker and, while stirring, the solutions A and B were simultaneously added thereto at a feed rate of about 25 cc/min, respectively. The pH of the resultant reaction mixture was 8.9. The stirring was further continued to effect the aging for one hour. The resultant cake was shifted with a sieve to obtain the metal (silver) salt of aluminum-containing silicate.

The results are shown in Table 2.

Examples 9 to 16

Various metal salts of aluminum-containing silicate containing nickel, cobalt, iron, titanium, barium, tin, or zirconium were prepared in the same manner as in Example 8 by using the solutions A and B listed in Table 1. The results are shown in Table 1.

Table 1

| Compound | Example No. | | | | | | | |
|---|---|---|---|---|---|---|---|---|
| | 9 | 10 | 11 | 12 | 13 | 14 | 15 | 16 |
| Solution A | | | | | | | | |
| #3 sodium silicate (mole $SiO_2$) | 273 g (1.0) | 273 g (1.0) | 273 g (1.0) | 273 g (1.0) | 273 g (1.0) | 273 g (1.0) | 273 g (1.0) | 273 g (1.0) |
| Sodium hydroxide (mole NaOH) | 60 g (1.5) | 60 g (1.5) | 60 g (1.5) | 60 g (1.5) | 60 g (1.5) | 60 g (1.5) | 60 g (1.5) | 60 g (1.5) |
| Solution B | | | | | | | | |
| Aluminium chloride · $6H_2O$ (mole $Al_2O_3$) | 145 g (0.3) | 145 g (0.3) | 145 g (0.3) | 145 g (0.3) | 145 g (0.3) | 145 g (0.3) | 145 g (0.3) | 145 g (0.3) |
| Cobalt chloride (mole CoO) | 65 g (0.5) | - | - | - | - | - | - | - |
| Nickel chloride · $6H_2O$ (mole NiO) | - | 120 g (0.5) | - | - | - | - | - | - |
| Ferric nitrate · $9H_2O$ (mole FeO) | - | - | 200 g (0.5) | - | - | - | - | - |
| Copper chloride (mole CuO) | - | - | - | 67 g (0.5) | - | - | - | - |
| Titanium sulfate (mole $TiO_2$) | - | - | - | - | 120 g (0.5) | - | - | - |
| Barium chloride · $2H_2O$ (mole BaO) | - | - | - | - | - | 122 g (0.5) | - | - |
| Tin sulfate · $2H_2O$ (mole SnO) | - | - | - | - | - | - | 126 g (0.5) | - |
| Zirconium chloride (mole $ZrO_2$) | - | - | - | - | - | - | - | 116.5 g (0.5) |

Example 17

A 109 g amount of #3 sodium silicate ($SiO_2$: 22.0%, $Na_2O$: 7.0%) and 94 g of sodium hydroxide (NaOH content: 2.35 mole) were dissolved in water to prepare 1 liter of a solution A ($SiO_2$ content: 0.4 mole).

On the other hand, 47 g of copper chloride (anhydrous salt), 43 g of zinc chloride (anhydrous salt) and 97 g of aluminum chloride (six hydrates) were dissolved in water to prepare 1 liter of a solution B (CuO content: 0.35 mole, ZnO content: 0.35 mole, $Al_2O_3$: 0.2 mole).

One liter of water was charged into a 5 liter beaker and, while stirring, the solutions A and B were simultaneously added thereto at a feed rate of about 25 cc/min, respectively. The pH of the resultant reaction mixture was 6.8 after the addition.

The stirring was further continued to effect the aging for one hour. The reaction mixture thus obtained was filtered with aspiration, followed by washing with water and drying at a temperature of 110° C. The cake thus obtained was sifted with a sieve to obtain metal (zinc, copper) salt of aluminum-containing silicate in the form of bluish white particles having a size of 8 to 16 mesh.

The results are shown in Table 2.

Comparative Example 1

To 120 g of commercially available hydrous finely divided silica (Mizukasil P-78 manufactured by Mizusawa Chemical Co.) ($SiO_2$ content: 2.0 mole), 80 g of water was added and the mixture was mixed or kneaded in a mortar, followed by drying at a temperature of 110°C and, further, calcining at a temperature of 500°C for 2 hours. The resultant cake was sifted with a sieve to obtain the white particles having a size of 8 to 16 mesh.

The results are shown in Table 2.

Comparative Example 2

A 114 g amount of commercially available zinc carbonate (manufactured by Masaoka Chemical Co.) (ZnO content: 1 mole) was kneaded with 80 g of water and, after drying at a temperature of 110°C, the mixture was calcined at a temperature of 500°C for 2 hours. The resultant cake was sifted with a sieve to obtain white particles having a size of 8 to 16 mesh.

The results are shown in Table 2.

Comparative Example 3

A 100 g amount of a commercially available silica-alumina catalyst ($SiO_2$ content: 66%, $Al_2O_3$ content: 21%) ($SiO_2$ content: 1.1 mole, $Al_2O_3$ content: 0.2 mole) was kneaded with 50 g of water and, after drying at a temperature of 110°C, the mixture was calcined at a temperature of 500°C for 2 hours. The resultant cake was sifted with a sieve to obtain white particles having a size of 8 to 16 mesh.

The composition ratios of the three components and the specific surface areas of the particle products obtained in Examples 1 to 17 and Comparative Examples 1 to 3 are shown in Table 2.

## Table 2

| No. | | $SiO_2$ (mole%) | $MO_{\frac{n}{2}}$ (mole%) | $Al_2O_3$ (mole%) | BET Specific Surface Area |
|---|---|---|---|---|---|
| Example | 1 | 31 | 54 | 15 | 210 |
| " | 2 | 41 | 38 | 21 | 205 |
| " | 3 | 40 | 60 | 0 | 145 |
| " | 4 | 33 | 56 | 11 | 218 |
| " | 5 | 35 | 51 | 14 | 133 |
| " | 6 | 50 | 34 | 16 | 290 |
| " | 7 | 60 | 40 | 0 | 206 |
| " | 8 | 67 | 13 | 20 | 216 |
| " | 9 | 55 | 28 | 17 | 185 |
| " | 10 | 55 | 28 | 17 | 162 |
| " | 11 | 55 | 28 | 17 | 158 |
| " | 12 | 55 | 28 | 17 | 163 |
| " | 13 | 55 | 28 | 17 | 171 |
| " | 14 | 55 | 28 | 17 | 160 |
| " | 15 | 55 | 28 | 17 | 154 |
| " | 16 | 55 | 28 | 17 | 172 |
| " | 17 | 31 | 54 | 15 | 177 |
| Comparative Example | 1 | 100 | – | – | 380 |
| " | 2 | – | 100 | – | 45 |
| " | 3 | 85 | – | 15 | 220 |

Evaluation of Deodorizing Effect

The following deodorizing amounts and deodorizing effects of the samples obtained in Examples 1 to 17 and Comparative Examples 1 to 3 as well as those of activated carbon were determined. The results are shown in Tables 3 and 4 below.

Deodorizing Amount Test Method (Forced Pass-Through Method)

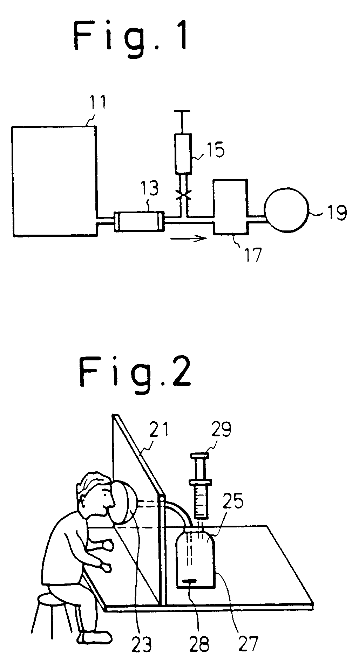

An apparatus as shown in Fig. 1 was used. A gas reservoir 11 was filled with air containing hydrogen sulfide and ammonia in a given amount shown below. The air was forcibly circulated through a column 13 packed with the deodorizer sample by means of a pump 19, while controlling a flow rate to 200 ml/min by a flow meter 17.

The gas volume was measured at the time when bad odors were detected in the outlet gas by a detecting tube 15. Thus, the deodorizing amount (mg/g) was calculated and represented as an index for a deodorizing amount or capability

| Hydrogen sulfide: | 10,000 ppm |
|---|---|
| Ammonia: | 5,000 ppm |

Deodorizing Effect Test Method (Organoleptic Evaluation)

As shown in Fig. 2, a partition screen 21 was stood on a base and a nose opening 23 for a smell bag was provided therein. A 1.8 liter bottle 27 used for mayonnaise was set at the back of the screen 21 and was connected via a soft plastic tube 25.

A 1 g amount of the deodorizer sample 28 was placed in a standarized bottle cap having a diameter of 4 cm so that the surface area of the deodorizer sample in each test was made equal and the bottle was sealed with a polyethylene cap provided with a gas inlet.

A bad odor was charged from the gas inlet so that the following predetermined gas concentrations were obtained. Initially set concentration

| ammonia: | 250 ppm |
|---|---|
| hydrogen sulfide: | 10 ppm |
| trimethylamine: | 1 ppm |
| ethylmercaptan: | 1 ppm |

The bottle was kept under room temperature for 6 hours and, as shown in Fig. 2, an injector 29 was vertically set to naturally fall down the piston. Thus, the air in the mayonnaise bottle 27 was discharged substantially at the same flow rate. The odor was evaluated under the following criteria by a panel consisting of five members. Of the evaluated points the highest and lowest points were cut and the remaining three evaluated points were averaged and the results were rounded to the nearest whole numbers.

Evaluation Criteria

| Score | Evaluation Criteria |
|---|---|
| 3 | Strong smell or odor |
| 2 | Kinds of smell or odor are apprehended (recognizable threshold concentration) |
| 1 | Smell is slightly detectable (detectable threshold concentration) |
| 0 | No smell |

The results are as follows.

Table 3

| No. | Deodorizing Amount (mg/g) | |
| --- | --- | --- |
| | Hydrogen Sulfide | Ammonia |
| Example 1 | 51.9 | 13.8 |
| Example 2 | 86.3 | 12.3 |
| Example 3 | 45.5 | 16.7 |
| Example 4 | 39.7 | 11.5 |
| Example 5 | 23.7 | 15.6 |
| Example 6 | 125.8 | 32.6 |
| Example 7 | 104.5 | 26.9 |
| Example 8 | 20.1 | 10.6 |
| Example 9 | 29.8 | 18.2 |
| Example 10 | 32.1 | 15.3 |
| Example 11 | 23.2 | 17.1 |
| Example 12 | 25.3 | 11.9 |
| Example 13 | 20.9 | 10.5 |
| Example 14 | 21.2 | 18.3 |
| Example 15 | 26.2 | 11.2 |
| Example 16 | 20.4 | 14.8 |
| Example 17 | 45.9 | 13.2 |
| Comparative Example 1 | 1.2 | 6.2 |
| Comparative Example 2 | 25.3 | 2.3 |
| Comparative Example 3 | 3.3 | 6.5 |
| Activated Carbon | 11.2 | 0.8 |

As is clear from the results shown above, the present deodorizer samples exhibit remarkably increased deodorizing effects when compared to the activated carbon.

Table 4

| No. | Deodorizing Effect (Evaluation Score) | | | |
|---|---|---|---|---|
| | Ethylmercaptan | Triethylamine | Hydrogen Sulfide | Ammonia |
| Example 1 | 1 | 0 | 0 | 0 |
| Example 2 | 0 | 0 | 0 | 0 |
| Example 3 | 0 | 0 | 0 | 0 |
| Example 4 | 1 | 0 | 0 | 0 |
| Example 5 | 0 | 0 | 0 | 0 |
| Example 6 | 0 | 0 | 0 | 0 |
| Example 7 | 0 | 0 | 0 | 0 |
| Example 8 | 1 | 1 | 0 | 0 |
| Example 9 | 1 | 0 | 0 | 0 |
| Example 10 | 0 | 0 | 0 | 0 |
| Example 11 | 0 | 0 | 0 | 0 |
| Example 12 | 0 | 0 | 0 | 0 |
| Example 13 | 1 | 0 | 1 | 0 |
| Example 14 | 0 | 0 | 0 | 0 |
| Example 15 | 1 | 0 | 0 | 0 |
| Example 16 | 1 | 0 | 1 | 0 |
| Example 17 | 0 | 0 | 0 | 0 |
| Comparative Example 1 | 3 | 2 | 3 | 1 |
| Comparative Example 2 | 1 | 3 | 0 | 2 |
| Comparative Example 3 | 3 | 2 | 3 | 1 |
| Activated Carbon | 1 | 1 | 0 | 2 |

The zinc salt of aluminum-containing silicate (i.e., zinc Al-silicate) was formulated, as a deodorizer, into various compositions. In each case, excellent deodorizing effects were observed.

Example 18: Enzyme Bleeching Agent

| Ingredient | wt% |
|---|---|
| Polyoxyethylene nonyl phenyl ether | 1 |
| Enzyme, alkaliprotease | 1 |
| Sodium percarbonate | 80 |
| Flavor | 0.3 |
| Zinc Al-silicate | 1 |
| Sodium carbonate | Balance |
| | 100% |

Example 19: Soap

| Ingredient | wt% |
|---|---|
| Soap base | 90 |
| Titanium dioxide | 0.5 |
| Flavor | 2 |
| Zinc Al-silicate | 0.5 |
| Purified water | Balance |
| | 100% |

Example 20: Powdery Bath Salt

| Ingredient | wt% |
|---|---|
| Sodium bicarbonate | 45 |
| Dry sodium sulfate | 42 |
| Flavor | 1.5 |
| Coloring agent | 0.5 |
| Zinc Al-silicate | 11 |
| | 100% |

Example 21: Dentifrice

| Ingredient | wt% |
|---|---|
| Glycerol | 25 |
| Sodium carboxy cellulose | 1.4 |
| Sodium benzoate | 0.5 |
| Silica | 34.0 |
| Sodium lauryl sulfate | 1.5 |
| Sodium monofluoro phosphate | 0.76 |
| Titanium dioxide | 0.4 |
| Zinc Al-silicate | 1.2 |
| Flavor | 1.0 |
| Purified water | Balance |
|  | 100% |

Example 22: Particle Detergent

| Ingredient | wt% |
|---|---|
| LAS-Na[*1] | 10 |
| AS-Na[*2] | 2 |
| AOS-Na[*3] | 10 |
| Zeolite (Type 4A) | 16 |
| Sodium silicate | 10 |
| Sodium carbonate | 10 |
| Zinc Al-silicate | 3.0 |
| Polyethylene glycol ($\overline{Mw}$ = 6000) | 1.0 |
| Tinopal CBS-X[*4] | 0.2 |
| Enzyme, Alkarase 2.0T[*5] | 0.4 |
| Water | 5 |
| Flavor | 0.3 |
| Sodium sulfate | Balance |
|  | 100% |

[*1] Sodium linear $C_{12}$ alkyl benzene sulfonate
[*2] Sodium $C_{12-16}$ alkyl sulfate
[*3] Sodium $C_{14}$-$C_{18}$ $\alpha$-olefin sulfonate
[*4] Fluorescent brightener (Chiba-Geigy)
[*5] Enzyme was powder blended after spray drying

Example 23: Hand Lotion

| Ingredient | wt% |
| --- | --- |
| Cetyl monostearate | 1.0 |
| Isopropyl palmitate | 4.0 |
| Polyethylene glycol 100 distearate | 2.0 |
| Zinc Al-silicate | 2.0 |
| Glycerol | 8.0 |
| Sodium cetyl sulfate | 5.0 |
| Manganese dioxide | 0.1 |
| Coloring agent, Flavor, Preservative | q.s. |
| Water | <u>Balance</u> 100% |

Example 24: Chewing Gum

| Ingredient | wt% |
| --- | --- |
| Gum base | 20 |
| Sugar | 16 |
| Isomaltose | 20 |
| Maltose | 20 |
| Corn syrup | 11.3 |
| Starch syrup | 11.5 |
| Flavor | 0.5 |
| Zinc Al-silicate | 0.5 |
| Hemoglobin | 0.2 |
| | 100% |

### Example 25: Liquid Cleanser

| Ingredient | wt% |
|---|---|
| Quartz sand (Ave. particle size 20µm) | 50 |
| Zinc Al-silicate | 10 |
| Sodium $C_{10}$-$C_{14}$ alkyl benzene sulfonate | 3.0 |
| Coconut fatty acid diethanolamide | 3.0 |
| Trisodium citrate | 3.0 |
| Sodium p-toluene sulfonate | 3.0 |
| Purified water | Balance |
| | 100% |

### Example 26: Toiletary Detergent

| Ingredient | wt% |
|---|---|
| Sodium polyacrylate (polymerization degree = 100,000) | 0.5 |
| Sodium polyoxyethylene ($\overline{p}$ = 3) $C_{12}$-$C_{14}$ alkylether sulfate | 0.2 |
| Zinc Al-silicate | 5 |
| Silica sand (Ave. particle size = 12 µm) | 8.0 |
| Ethylene glycol | 2.0 |
| 1,2-Benzisothiazoline-3-one | 0.2 |
| Limonen | 0.03 |
| Water | Balance |
| | 100% |

### Example 27

A 1.3 g amount of zinc Al-silicate was filled, together with 48.7 g of fron gas (3:7 mixture of Fron 11 and Fron 12), into a container to prepare a small sized powder spray. This powder spray suppressed axilla smell or tragomaschalia when sprayed on the axilla.

### Example 28

The comparative deodorizing tests using the present deodorizer and comparative deodorizers were carried out with respect to four components, ethylmercaptan, hydrogen sulfide, ammonia, and methylamine, which represent the menstrual smell. The deodorizing test was carried out as follows. The results are shown in Table 5.

Deodorizing Test

A 0.1 g amount of the sample was charged into a 500 ml injector and one type of a smelling liquid was dropwise added to the approximately center portion of the sample from the open mouth of the injector by a micro syringe, followed by immediately plugging the open mouth. The injector was laid for one hour in a constant temperature and constant humidity vessel at 35°C and 85% R.H. The injector was shaken every 30 minutes, whereby the inside air was mixed.

Thereafter, one end of a gas detecting tube was corrected to a rubber tube provided at the opening mouth of the injector and the other end was attached to a gas detector (i.e., Kitagawa type model AP-1 or No. 400). Thus, the gas concentration A was determined.

On the other hand, three piled Toyo filter papers (No. 2, 55 mm diameter), instead of the sample, were placed in the 500 ml injector. The smelling liquid was dropwise added in the same manner as mentioned above. Thus, the gas concentration B (i.e., blank value) was determined in the same manner as mentioned above.

The deodorizing efficiency was determined as follows.

$$\text{Deodorizing efficiency (\%)} = (1 - \frac{A}{B}) \times 100$$

The results are shown in Table 5. In Table 5, the zinc aluminosilicate was white powder having a composition, in terms of a mole ratio, of $ZnO:Al_2O_3:SiO_2 = 50:20:30$ and the polymer absorbent was white powder comprising crosslinked product of sodium polyacrylate. In the above-mentioned deodorizing tests, a mixture of 40 parts by weight of the zinc aluminosilicate and 100 parts by weight of the polymer absorbent was used as a sample according to the present invention. On the other hand, the magnesium aluminosilicate used as a comparative sample in this test was a white powder having a composition of $MgO:Al_2O_3:SiO_2 = 30:40:30$ (mole ratio).

Table 5

| Run No. | Sample | Deodorizing Efficiency (%) | | | |
|---|---|---|---|---|---|
| | | Ethylmercaptan | Hydrogen sulfide | Ammonia | Methyl amine |
| 1 | Zn alumino silicate + Polymer absorbent | 92 | 99 | 90 | 83 |
| 2* | Mg alumino silicate + Polymer absorbent | 35 | 70 | 85 | 76 |
| 3* | Zn alumino silicate | 90 | 99 | 80 | 82 |
| 4* | Polymer absorbent | 0 | 0 | 87 | 76 |
| | Blank Smell Conc. (ppm) | (60) | (90) | (625) | (150) |

\* Comparative Example

Example 29

Sanitary napkins each comprising a laminate obtained by laminating, from the surface side, a rayon staple cotton fiber layer (30 g/m²), a pulp layer (250 g/m²), each deodorant layer (0.3 g) listed in Table 6, and an absorbing paper layer (a thin paper sheet (30 g/m²) x 3 sheets) were prepared. The sanitary napkin was covered with a polyethylene laminated paper except for the surface thereof, followed by entirely wrapping with non-woven cloth (16 g/m²). In the samples used in Run Nos. 1 and 2, the deodorizer layer was composed of the two layer structure, i.e., the copper polymer absorbent layer and the lower inorganic powder layer.

Thereafter, the napkin prepared above was placed in a 1 liter bottle and smelling or odoring substances were dropped thereinto in a given amount by a microsyringe, thereafter, the bottle was laid for one hour under the conditions of 35°C and 85°C R.H. in a constant temperature and constant humidity bath. Thereafter, the existence of an odor was determined an expert panel composed of five members. The results are determined according to the following evaluation standard.

(-): No odor
(±): Slight odor
(+) : Strong odor

The results are shown in Table 6.

Table 6

| Run No. | Sample | Deodorizing Efficiency (%) | | | |
|---|---|---|---|---|---|
| | | Ethylmercaptan | Hydrogen sulfide | Ammonia | Methyl amine |
| 1 | Zn alumino silicate (40 parts) + Polymer absorbent (100 parts) | - | - | - | - |
| 2* | Mg alumino silicate (40 parts) + Polymer absorbent (100 parts) | + | ± | - | ± |
| 3* | Zn alumino silicate | - | - | ± | - |
| 4* | Polyr absorbent | + | + | - | -~± |
| 5* | Activated carbon | - | - | + | + |
| | Blank Smell Conc. (ppm) | (60) | (90) | (625) | (150) |

\* Comparative Example

Example 30

Zinc aluminosilicate having a composition of 31 mole% $SiO_2$, 54 mole% ZnO, and 15 mole% $Al_2O_3$ was obtained in the form of white powder in the same manner as in Example 1. The Hunter's whileness of the resultant powder was 95%, as determined by means of Automatic Refractometer Model TR-600 manufactured by Tokyo Denshoku.

The deodorizing power of this powder was evaluated as follows.

Into a 20 liter polyethylene bucket, 10 liters of water and 50 g of pulp were charged and uniformly dispersed, while stirring, to prepare a liquid A.

On the other hand, 30 g of zinc aluminosilicate obtained above was charged into a 10 liter polyethylene bucket, followed by dispersing in water. Thus, a liquid B was prepared.

The liquied B was added, while stirring, to the liquid A to adjust the final volume to 16 liters. From 0.75 liter of the resultant mixture, a sheet was made by using a standard hand paper making machine (manufactured by Toyo Seiki Co.). Thus, a deodorizing sheet was prepared. The content of the zinc aluminosilicate was about 25% after air drying.

The resultant deodorizing sheet obtained above was white and excellent in the visual appearance thereof and the zinc aluminosilicate was firmly supported.

The deodorizing sheet was then cut to a size of 5 cm × 5 cm and used as sample sheets for determining the deodorizing power against offensive or unpleasant odors as follows.

That is, a sample deodorizing sheet and 5 ml of 28% aqueous ammonia (i.e., initial ammonia concentration = 1000 ppm) were placed in a 1.8 liter bottle used for mayonnaise and the ammonia concentration after the elapse of a given time was measured. The results are shown in Table 7.

As a Comparative Example, a sample sheet having the same size composed of activated carbon and fiber was evaluated. The results are also shown in Table 7.

Table 7

| Ammonia Concentration (ppm) | | |
|---|---|---|
| Time | Example | Comparative Example |
| after 30 min. | 75 | 650 |
| after 1 hr | 5 | 500 |
| after 2 hrs | Not detected | 430 |

Example 31

Zinc aluminosilicate having a composition of 41 mole% $SiO_2$, 38 mole% ZnO, and 21 mole% $Al_2O_3$ was obtained in the form of white powder in the same manner as in Example 2. The Hunter's whileness of the resultant powder was 88%, as determined by means of, Automatic Refractometer Model TR-600 manufactured by Tokyo Denshoku.

The deodorizing power of this powder was evaluated as follows.

Into a 20 liter polyethylene bucket, 10 liters of water and 70 g of pulp were charged and uniformly dispersed, while stirring, to prepare a liquid A.

On the other hand, 80 g of zinc aluminosilicate obtained above was charged into a 10 liter polyethylene bucket, followed by dispersing in water. Thus, a liquid B was prepared.

The liquid B was added, while stirring, to the liquid A to against the final volume to 16 liters. From 0.8 liters of the resultant mixture, a sheet was made by using a standard hand paper making machine (manufactured by Toyo Seiki Co.). Thus, a deodorizing sheet was prepared. The content of the zinc aluminosilicate was about 50% after air drying.

When the deodorizing sheet was placed at a triangle corner of a sink, not only the unpleasant odor derived from leftover meal was effectively suppressed, but also the slimy or blackish phenomemon at the corner disappeared.

## Claims

1. A method of making a deodoriser comprising, as an effective component, at least one compound selected from metal silicates and metal salts of aluminum-containing silicates having a following composition, in terms of their oxides:

| | |
|---|---|
| $SiO_2$: | 5 to 80 mole% |
| $MOn_{/2}$; | 5 to 65 mole% |
| $Al_2O_3$: | 0 to 60 mole% |

wherein M represents at least one metal selected from zinc, copper, silver, cobalt, nickel, iron, titanium, tin and zirconium, and $\underline{n}$ is the valency of the metal, said method including the step of preparing said metal silicates and metal salts of aluminum-containing silicates by reacting in the presence of water a water-soluble silicate and water-soluble metal salt and, when $Al_2O_3$ is present, a water soluble aluminum salt and/or a water soluble aluminate, in a ratio corresponding to the above-mentioned composition ratio.

2. A method according to claim 1 wherein the said metal silicate or metal salt of an aluminum-containing silicate is prepared so as to have a specific surface area of 100 $m^2$/g or more determined according to a BET method.

3. Sanitary goods comprising a deodorizer when made according to any preceding claim, in the form of an inorganic white powder, and a polymer absorbent powder as a liquid absorbent, and wherein M is zinc and is present in an amount of 5 to 60 mole% in terms of its oxide ZnO.

4. Sanitary goods as claimed in claim 3, wherein the amount of the inorganic white powder is 5 to 80 parts by weight, based on 100 parts by weight of the polymer absorbent powder.

5. A deodorizing sheet comprising a deodoriser when made according to any one of claims 1 or 2, wherein M is zinc and said composition comprises at least 1 mole% $Al_2O_3$.

## Patentansprüche

1. Verfahren zur Herstellung eines Desodoriermittels mit mindestens einer Verbindung aus der Gruppe Metallsilikate und Metallsalze aluminiumhaltiger Silikate folgender, in Oxidform ausgedrückter Zusammensetzung:

| | |
|---|---|
| $SiO_2$: | 5 bis 80 Mol% |
| MO n/2: | 5 bis 65 Mol% |
| $Al_2O_3$: | 0 bis 60 Mol% |

worin bedeuten:
M mindestens ein Metall aus der Gruppe Zink, Kupfer, Silber, Kobalt, Nickel, Eisen, Titan, Zinn und Zirkon und n die Valenz eines Metalls,
als wirksamer Komponente, bei welchem die Metallsilikate und Metallsalze aluminiumhaltiger Silikate durch Umsetzen eines wasserlöslichen Silikats und wasserlöslichen Metallsalzes und, bei Vorhandensein von $Al_2O_3$, eines

wasserlöslichen Aluminiumsalzes und/oder wasserlöslichen Aluminats in einem dem obigen Zusammensetzungsverhältnis entsprechenden Verhältnis in Gegenwart von Wasser hergestellt werden.

2. Verfahren nach Anspruch 1, wobei das Metallsilikat oder synthetische Metallsalz eines aluminiumhaltigen Silikats derart hergestellt wird, daß es eine nach der BET-Methode bestimmte spezifische Oberfläche von 100 m²/g erhält.

3. Hygieneartikel mit nach einem der vorhergehenden Ansprüche hergestellten Desodoriermittel in Form eines anorganischen weißen Pulvers und
einem polymeren Absorptionsmittelpulver als Flüssigkeits-Absorptionsmittel, wobei M für Zink steht und in einer Menge von 5 bis 60 Mol%, ausgedrückt als sein Oxid ZnO, vorliegt.

4. Hygieneartikel nach Anspruch 3, wobei die Menge an dem anorganischen weißen Pulver, bezogen auf 100 Gew.-Teile des polymeren Absorptionsmittelpulvers, 5 bis 80 Gew.-Teile beträgt.

5. Desodorierfolie oder -lage mit einem nach einem der Ansprüche 1 und 2 hergestellten Desodoriermittel, wobei M für Zink steht und das Mittel mindestens 1 Mol% $Al_2O_3$ enthält.

**Revendications**

1. Procédé de fabrication d'un désodorisant comprenant comme ingrédient actif au moins un compose choisi parmi des silicates métalliques et des sels métalliques de silicates contenant de l'aluminium ayant la composition suivante, exprimée par leurs oxydes :

| | |
|---|---|
| $SiO_2$ : | 5 à 80 moles % |
| $Mon/_2$ : | 5 à 65 moles % |
| $Al_2O_3$ : | 0 à 60 moles % |

dans laquelle M représente au moins un métal choisi parmi le zinc, le cuivre, l'argent, le cobalt, le nickel, le fer, le titane, l'étain et le zirconium, et n est la valence du métal, ce procédé comprenant l'étape de préparation de ces silicates métalliques et de ces sels métalliques de silicate contenant de l'aluminium en faisant réagir en présence d'eau un silicate soluble dans l'eau et un sel métallique soluble dans l'eau et, lorsque $Al_2O_3$ est présent, un sel d'aluminium soluble dans l'eau et/ou un aluminate soluble dans l'eau, dans un rapport correspondant au rapport de composition mentionné ci-dessus.

2. Procédé selon la revendication 1, dans lequel ledit silicate métallique ou ledit sel métallique d'un silicate contenant de l'aluminium est préparé de manière à avoir une surface spécifique, déterminée par la méthode BET, de 100 m²/g ou davantage.

3. Article sanitaire comprenant un désodorisant fabriqués selon l'une quelconque des revendications précédentes, sous la forme d'une poudre blanche minérale et d'une poudre absorbante de polymère comme absorbant des liquides, et dans lequel M est le zinc et est présent dans une proportion, exprimée par son oxyde de ZnO, de 5 à 60 moles %.

4. Article sanitaire selon la revendication 3, dans lequel la proportion de poudre blanche minérale est de 5 à 80 parties en poids pour 100 parties en poids de la poudre absorbante de polymère.

5. Feuille désodorisante comprenant un désodorisant fabriqué selon l'une quelconque des revendications 1 ou 2, dans laquelle M est le zinc, et ladite composition comprend au moins 1 mole % d'$Al_2O_3$.

# Fig. 1

# Fig.2